(19) Europäisches Patentamt
European Patent Office
Office européen des brevets

(11) EP 0 990 442 A1

(12) # EUROPEAN PATENT APPLICATION

(43) Date of publication:
**05.04.2000 Bulletin 2000/14**

(51) Int. Cl.[7]: **A61K 31/40**

(21) Application number: **98309616.5**

(22) Date of filing: **24.11.1998**

(84) Designated Contracting States:
**AT BE CH CY DE DK ES FI FR GB GR IE IT LI LU MC NL PT SE**
Designated Extension States:
**AL LT LV MK RO SI**

(30) Priority: **26.11.1997 US 66555 P**

(71) Applicant: **ELI LILLY AND COMPANY**
**Indianapolis, Indiana 46285 (US)**

(72) Inventors:
- **Jirousek, Michael R.**
  **Abbott Park, Illinois 60064-3500 (US)**
- **Ways, Douglas Kirk**
  **Indianapolis, Indiana 46205 (US)**
- **Ballas, Lawrence M.**
  **Apex, North Carolina 27502 (US)**
- **Stramm, Lawrence E.**
  **Indianapolis, Indiana 46256 (US)**

(74) Representative:
**Harrison, Ivor Stanley et al**
**Withers & Rogers**
**Goldings House**
**2 Hays Lane**
**London SE1 2HW (GB)**

(54) **Therapeutic treatment for chronic myeloid leukemia and active lymphoid leukemia**

(57) A method for treating neoplasms associated with an oncogenic form of ABL such as CML and ALL is disclosed, particularly using the isozyme selective PKC inhibitor, (S)-3,4-[N, N'-1,1'-((2''-ethoxy)-3'''(O)-4'''-(N,N-dimethylamino)-butane)-bis-(3,3'-indolyl)]-1(H)-pyrrole-2,5-dione and it pharmaceutically acceptable salts.

EP 0 990 442 A1

## Description

### BACKGROUND OF THE INVENTION

1. Field of the Invention

The present invention is broadly directed to a method for inducing apoptosis or cell death. The present invention is particularly directed to the use of a particular class of β isozyme selective Protein Kinase C (PKC) inhibitors for treating neoplasms associated with oncogenic forms of the Aberson leukemia (*ABL*) gene, *e.g.*, chronic myeloid leukemia (CML) and acute lymphoid leukemia (ALL).

2. Description of Related Art

Cell growth and cell death are controlled by various factors through a complex molecular network. One of the causes for neoplasia is defect(s) in the apoptosis pathway or in the process of programed cell death. Neoplastic cells no longer cease multiplication, in other words such cells fail to go through cell death when they reach a critical mass, and the uncontrolled cell growth leads to severe consequences, *e.g.*, metastasis, and the death of the host.

Studies have shown that oncogenic forms of non-receptor tyrosine kinase ABL are associated with neoplasms via suppression of apoptosis. One of the oncogenic forms of ABL is derived from a chromosome rearrangement. For instance, in chronic myeloid leukemia (CML), a chromosomal translocation commonly occurs resulting in chromosome [(t 9:22), (q34, q11)] which is termed the Philadelphia chromosome (Nowell P. And Hungerford D., et al 1960. Science 132:1497). This chromosomal rearrangement disrupts the normal function of the *ABL* gene and produces a chimeric gene product (BCR-ABL) implicated in the pathogenesis of CML (Daley G., et al., 1990. Science 247: 824; Elefanty A, et al., 1990. EMBO J 9: 1069; Kelliher M, et al., 1990. Proc Natl Acad Sci USA 87: 6649). A similar chromosomal rearrangement and chimeric gene product also exist in certain cases of acute lymphoid leukemia (ALL) (Kurzrock R., et al., 1988. 319: 990; Berger R., et al., 1990. Cancer Genet Cytogenet 4: 143). Viral ABL (v-ABL) is another oncogenic form. It can cause murine leukemia. Studies have shown that administering a non-specific protein kinase C inhibitor, calphostin C to the cells containing v-ABL induces apoptosis and cell death (Evans C., et al., 1995. J Cell Sci 108: 2591, Evans C., et al., 1995. J Cell Sci 108: 2591). However, because calphostin C has to be light activated and is not isozyme selective, it cannot be applied clinically.

Therapeutic treatments have been developed over the years to treat neoplasms. However, there is still a need in the art to identify new therapeutic agents for cancer treatment, especially therapeutic agents targeted at the apoptosis pathway and the factors associated therewith.

### SUMMARY OF INVENTION

It is an object of the invention to provide methods for treating a neoplasm associated with an oncogenic form of ABL.

It is another object of the invention to provide methods for treating chronic myeloid leukemia associated with an oncogenic form of ABL.

It is yet another object of the invention to provide methods for treating acute lymphoid leukemia associated with an oncogenic form of ABL.

It is still another object of the invention to provide a method for inducing apoptosis in cells.

These and other objects of the invention are provided by one or more of the embodiments described below.

In one embodiment of the invention, there is provided a method for treating a neoplasm associated with an oncogenic form of ABL which comprises administrating to a mammal in need of such treatment a therapeutically effective amount of an inhibitor of the β isozyme of protein kinase C.

In still another embodiment of the invention, there is provided a method for treating chronic myeloid leukemia (CML) associated with an oncogenic form of ABL which comprises administrating to a mammal in need of such treatment a therapeutically effective amount of an inhibitor of the β isozyme of protein kinase C.

In another embodiment of the invention, there is provided a method for treating acute lymphoid leukemia (ALL) associated with an oncogenic form of ABL which comprises administrating to a mammal in need of such treatment a therapeutically effective amount of an inhibitor of the β isozyme of protein kinase C.

In another embodiment of the invention there is provided a method for inducing apoptosis in cells which comprises contacting said cells with an apoptosis inducing amount of an inhibitor of the β isozyme of protein kinase C.

The present invention provides the art with a method for inducing apoptosis in cells and is effective in treating neoplasms associated with an oncogenic form of ABL, *e.g.*, CML and ALL.

## DETAILED DESCRIPTION OF THE INVENTION

It is a discovery of the present invention that use of a particular class of β-isozyme selective protein kinase C inhibitors induces apoptosis or cell death. Consequently, such compounds can be used therapeutically to treat neoplasms associated with oncogenic forms of ABL, especially the oncogenic forms of ABL involved in suppression of apoptosis.

Protein kinase C exists as a gene family consisting of at least twelve members of isoforms including the conventional isoforms, *i.e.*, α, β-1, β-2, and γ, and novel isoforms, *i.e.*, δ, ε, θ, and η. The method of this invention preferably utilizes those protein kinase C inhibitors that effectively inhibit the β isozyme, *e.g.*, β-1 and β-2 isozymes.

One suitable group of compounds are generally described in the prior art as bis-indolylmaleimides or macrocyclic bis-indolylmaleimides. Bis-indolylmaleimides are well recognized in the prior art include those compounds described in U.S. Patents 5621098, 5552396, 5545636, 5481003, 5491242, and 5057614, all incorporated herein by reference. Macrocyclic bis-indolylmaleimides are particularly represented by the compounds of formula I. These compounds, and methods for their preparation, have been disclosed in U. S. Patent 5,552,396, which is incorporated herein by reference. These compounds are administered in a therapeutically effective amount to a mammal to induce apoptosis or cell death. In particular, these compounds can be used to inhibit the effects of oncogenic forms of ABL and treat neoplasms which stem from the oncogenic forms of ABL, *e.g.*, CML and ALL.

One preferred class of compounds for use in the method of the invention has the formula:

**(I)**

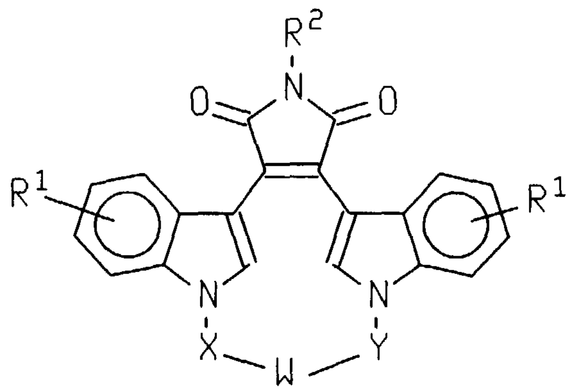

wherein:

W is -O-, -S-, -SO-, $-SO_2-$, -CO-, $C_2$-$C_6$ alkylene, substituted alkylene, $C_2$-$C_6$ alkenylene, -aryl-, $-aryl(CH_2)_mO-$, -heterocycle-, $-heterocycle-(CH_2)_mO-$, -fused bicyclic-, $-fused bicyclic-(CH_2)_mO-$, $-NR^3-$, $-NOR^3-$, -CONH-, or -NHCO-;

X and Y are independently $C_1$-$C_4$ alkylene, substituted alkylene, or together X, Y, and W combine to form $-(CH_2)_n-$AA-;

$R^1$s are hydrogen or up to four optional substituents independently selected from halo, $C_1$-$C_4$ alkyl, hydroxy, $C_1$-$C_4$ alkoxy, haloalkyl, nitro, $NR^4R^5$, or - NHCO($C_1$-$C_4$ alkyl);

$R^2$ is hydrogen, $CH_3CO$-, $NH_2$, or hydroxy;

$R^3$ is hydrogen, $(CH_2)_m$aryl, $C_1$-$C_4$ alkyl, -COO($C_1$-$C_4$ alkyl), $-CONR^4R^5$, - $(C{=}NH)NH_2$, -SO($C_1$-$C_4$ alkyl), $-SO_2$ $(NR^4R^5)$, or $-SO_2$ ($C_1$-$C_4$ alkyl);

$R^4$ and $R^5$ are independently hydrogen, $C_1$-$C_4$ alkyl, phenyl, benzyl, or combine to the nitrogen to which they are bonded to form a saturated or unsaturated 5 or 6 member ring;

AA is an amino acid residue;

m is independently 0, 1, 2, or 3; and

n is independently 2, 3, 4, or 5.

A more preferred class of compounds for use in this invention is represented by formula I wherein the moieties -X-W-Y- contain 4 to 8 atoms, which may be substituted or unsubstituted. Most preferably, the moieties -X-W-Y- contain 6 atoms.

Other preferred compounds for use in the method of this invention are those compounds of formula I wherein $R^1$ and $R^2$ are hydrogen; and W is a substituted alkylene, -O-, S-, -CONH-, -NHCO- or $-NR^3-$. Particularly preferred compounds are compounds of the formula Ia:

(Ia)

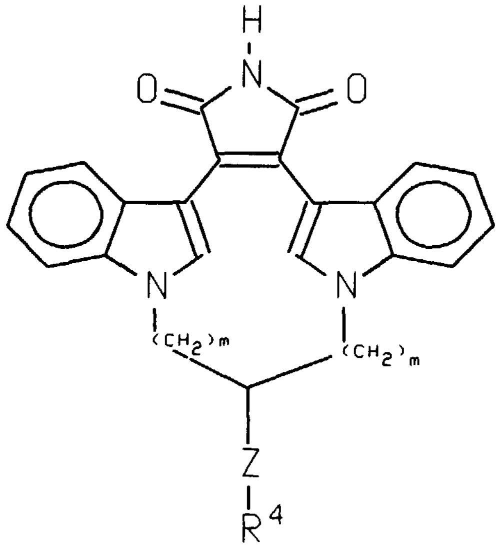

wherein Z is $-(CH_2)_p-$ or $-(CH_2)_p-O-(CH_2)_p-$; $R^4$ is hydroxy, -SH, $C_1$-$C_4$ alkyl, $(CH_2)_m$aryl, -NH(aryl), $-N(CH_3)(CF_3)$, $-NH(CF_3)$, or $-NR^5R^6$; $R^5$ is hydrogen or $C_1$-$C_4$ alky; $R^6$ is hydrogen, $C_1$-$C_4$ alkyl or benzyl; p is 0, 1, or 2; and m is independently 2 or 3. Most preferred compounds of the formula Ia are those wherein Z is $CH_2$; and $R^4$ is $-NH_2$, $-NH(CF_3)$, or $-N(CH_3)_2$.

Other preferred compounds for use in the method of the present invention are compounds wherein W in formula I is -O-, Y is a substituted alkylene, and X is an alkylene. These preferred compounds are represented by formula Ib:

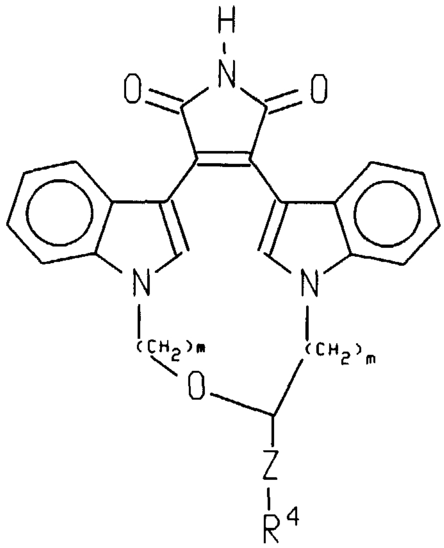

(Ib)

wherein Z is $-(CH_2)_p-$; $R^4$ is $-NR^5R^6$, $-NH(CF_3)$, or $-N(CH_3)(CF_3)$; $R^5$ and $R^6$ are independently H or $C_1$-$C_4$ alkyl; p is 0, 1, or 2; and m is independently 2 or 3. Most preferred compounds of formula Ib are those wherein p is 1; and $R^5$ and $R^6$ are methyl.

Because they contain a basic moiety, the compounds of formula I, Ia, and Ib can also exist as pharmaceutically acceptable acid addition salts. Acids commonly employed to form such salts include inorganic acids such as hydrochloric, hydrobromic, hydroiodic, sulfuric and phosphoric acid, as well as organic acids such as para-toluenesulfonic, methanesulfonic, oxalic, para-bromophenylsulfonic, carbonic, succinic, citric, benzoic, acetic acid, and related inorganic and organic acids. Such pharmaceutically acceptable salts thus include sulfate, pyrosulfate, bisulfate, sulfite, bisulfite, phosphate, mono-hydrogenphosphate, dihydrogenphosphate, metaphosphate, pyrophosphate, chloride, bromide, iodide, acetate, propionate, decanoate, caprylate, acrylate, formate, isobutyrate, heptanoate, propiolate, oxalate, malonate, succinate, suberate, sebacate, fumarate, maleate, 2-butyne-1,4-dioate, 3-hexyne-2, 5-dioate, benzoate, chlorobenzoate, hydroxybenzoate, methoxybenzoate, phthalate, xylenesulfonate, phenylacetate, phenylpropionate, phenylbutyrate, citrate, lactate, hippurate, β-hydroxybutyrate, glycolate, maleate, tartrate, methanesulfonate, propanesulfonate, naphthalene-1-sulfonate, naphthalene-2-sulfonate, mandelate and the like. Particularly the hydrochloric and mesylate salts are used.

In addition to pharmaceutically-acceptable salts, other salts also can exist. They may serve as intermediates in the purification of the compounds, in the preparation of other salts, or in the identification and characterization of the compounds or intermediates.

The pharmaceutically acceptable salts of compounds of formulae I, Ia, and Ib can also exist as various solvates, such as with water, methanol, ethanol, dimethylformamide, ethyl acetate and the like. Mixtures of such solvates can also be prepared. The source of such solvate can be from the solvent of crystallization, inherent in the solvent of preparation or crystallization, or adventitious to such solvent.

It is recognized that various stereoisomeric forms of the compounds of formulae I, Ia, and Ib may exist; for example, W may contain a chiral carbon atom in the substituted alkylene moiety. The compounds are normally prepared as racemates and can conveniently be used as such. Alternatively, both individual enantiomers can be isolated or synthesized by conventional techniques if so desired. Such racemates and individual enantiomers and mixtures thereof form part of the compounds used in the methods of the present invention.

The compounds utilized in this invention also encompass the pharmaceutically acceptable prodrugs of the compounds of formulae I, Ia, and Ib. A prodrug is a drug which has been chemically modified and may be biologically inactive at its site of action, but which may be degraded or modified by one or more enzymatic or other *in vivo* processes to the parent bioactive form. This prodrug likely may have a different pharmacokinetic profile than the parent, enabling easier absorption across the mucosal epithelium, better salt formation or solubility, and/or improved systemic stability (an increase in plasma half-life, for example). Typically, such chemical modifications include the following:

1) ester or amide derivatives which may be cleaved by esterases or lipases;
2) peptides which may be recognized by specific or nonspecific proteases; or
3) derivatives that accumulate at a site of action through membrane selection of a prodrug form or a modified prodrug form; or any combination of 1 to 3, <u>supra</u>. Conventional procedures for the selection and preparation of suitable prodrug derivatives are described, for example, in H. Bundgaard, <u>Design of Prodrugs</u>, (1985).

The synthesis of various bis-indole-N-maleimide derivatives is described in Davis *et al.* U.S. Patent 5,057,614 and the synthesis of the preferred compounds suitable for use in this invention are described in the previously identified U.S. Patent 5,552,396 and 5,057,614 and in Faul *et al.* EP publication 0 657 411 A1, both of which are incorporated herein by reference.

One particularly preferred protein kinase C inhibitor for use in the method of this invention is the compound described in Example 5g ((S)-3,4-[N, N'-1,1'-((2''-ethoxy)-3'''(O)-4'''-(N,N-dimethylamino)-butane)-bis-(3,3'-indolyl)]-1(H)-pyrrole-2,5-dione Hydrochloride Salt) of the aforementioned U.S. Patent 5,552,396. This compound is a potent protein kinase C inhibitor. It is selective to protein kinase C over other kinases and is highly isozyme-selective, i.e., it is selective for the beta-1 and beta -2 isozymes. Other salts of this compound also would be favored, especially the mesylate salts.

A preferred mesylate salt can be prepared by reacting a compound of the formula II

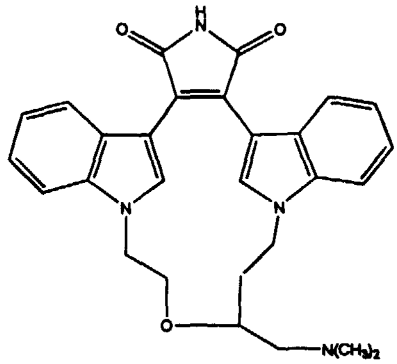

(II)

with methanesulfonic acid in a non-reactive organic solvent, preferably an organic/water mixture, and most preferably water-acetone. Other solvents such as methanol, acetone, ethylacetate and mixtures thereof are operable. The ratio of solvent to water is not critical and generally determined by the solubility of the reagents. Preferred solvent to water ratios are generally from 0.1:1 to 100:1 solvent to water by volume. Preferably, the ratio is 1:1 to 20:1 and most preferably 5:1 to 10:1. The optimal ratio is dependent on the solvent selected and is preferably acetone at a 9:1 solvent to water ratio.

The reaction usually involves approximately equimolar amounts of the two reagents, although other ratios, especially those wherein the methanesulfonic acid is in excess, are operative. The rate of addition of methanesulfonic acid is not critical to the reaction and may be added rapidly (<5 minutes) or slowly over 6 or more hours. The reaction is carried out at temperatures ranging from 0°C to reflux. The reaction mixture is stirred until formation of the salt is complete, as determined by x-ray powder diffraction and can take from 5 minutes to 12 hours. The salts of the present invention are preferably and readily prepared as a crystalline form. The trihydrate form of the salt may be readily converted to the monohydrate upon drying or exposure to 20-60% relative humidity. The salt is substantially crystalline demonstrating a defined melting point, birefringence, and an x-ray diffraction pattern. Generally, the crystals have less than 10% amorphous solid and preferably less than 5% and most preferably less than 1% amorphous solid.

The mesylate salt is isolated by filtration or other separation techniques appreciated in the art directly from the reaction mixture in yields ranging from 50% to 100%. Recrystallization and other purification techniques known in the art may be used to further purify the salt if desired.

Oncogenic forms of ABL include those protein products which are derived from wild-type ABL and have oncogenic effects *in vitro* or *in vivo.* Oncogenic effects include but are not limited to neoplasia, cell transformation, and any other activities known in the art that are characteristic of neoplasia. Oncogenic forms of ABL can be protein factors that contain a portion or portions of ABL, or have sequence similarity to ABL. For example, one kind of oncogenic form of ABL is a viral ABL (v-ABL). The v-ABL is a constitutively activated form of the non-receptor tyrosine kinase ABL. When it is expressed in mice, it causes murine leukemia. Another kind of oncogenic form of ABL is a fusion protein or a chimeric protein resulting or stemming from chromosome rearrangement, *e.g.*, BCR-ABL. The fusion proteins or chimeric proteins produced by chromosome rearrangement, cause a type of murine leukemia similar to that induced by the v-ABL in mice (Kelliher M, et al., 1990. Proc Natl Acad Sci USA 87: 6649).

Several neoplasms are known to be associated with oncogenic forms of ABL. For instance, in chronic myeloid leukemia (CML), a chromosome translocation [(t 9:22), (q34, q110] results in a chimeric chromosome termed the Philadelphia chromosome (Nowell P. And Hungerford D., et al., 1960. Science 132:1497). Such chromosome rearrangement disrupts the normal reading frame of the *ABL* gene and produces a chimeric or fusion gene product, BCR-ABL (Daley G., et al., 1990. Science 247:824; Elefanty A, et al., 1990. EMBO J 9:1069; Kelliher M, et al., 1990. Proc Natl Acad Sci USA 87:6649). A similar chromosomal rearrangement and fusion protein product exist in cases of acute lymphoid leukemia (ALL) (Kurzrock R., et al., 1988. 319:990; Berger R, et al., 1990. Cancer Genet Cytogenet 4:143).

Studies show that oncogenic forms of ABL activate subfamilies of protein kinase C (PKC) which correlate with the suppression of apoptosis. Expression of v-*ABL* gene increases intracellular levels of diacylglycerol which is an intracellular activator of protein kinase C subfamilies including conventional subfamilies, *e.g.*, $\alpha$, $\beta$-1, $\beta$-2 and $\gamma$ isoforms, and novel subfamilies, *e.g.*, $\delta$, $\varepsilon$, $\theta$, and $\eta$ isoforms (Dive C., et al., 1993. Pro Amer Assoc Cancer Res 34: 516; Owen P., et al., 1993. J Biol Chem 268: 15696; Hug H and Sarre T, 1993. Biochem J 291: 329). Expression of v-*ABL* in interleukin-3-dependent hematopoietic cells also results in translocation of protein kinase C $\beta$-2 isoform to the nucleus and suppression of apoptosis (Evans C., et al., 1995. J Cell Sci 108: 2591).

Applicants believe that inhibition of a protein kinase C isoform, *e.g.*, PKC-$\beta$, by the compounds identified by

the present invention will induce apoptosis in cells and specifically restore or reinduce apoptosis suppressed by oncogenic forms of ABL in hematopoietic cells.

Apoptosis is a specific mode of cell death recognized by a characteristic pattern of morphological, biochemical, and molecular changes including but not limited to, endonucleolysis (DNA ladder), abnormal DNA breaks, and condensation of chromatin and cytoplasm (condensed and punctuated nuclei). These changes can be readily detected by any means known in the art, *e.g.*, microscopy, flow cytometric methods based on increased sensitivity of DNA to denaturation, and altered light scattering properties, DNA fragmentation as assessed by agarose gel electrophoresis, terminal DNA transferase assay, (TdT assay), and nick translation assay (NT assay).

Inhibited or suppressed apoptosis or cell death is known to be involved in the pathogenesis of neoplasms, *e.g.*, BCR-ABL suppresses apoptosis or cell death of hematopoietic cells and induces the chronic and blast phases of CML (Evans C., et al., 1993. Cancer Res 53: 1735). Therefore, the compounds identified and described in the present invention can be used to treat neoplasms associated with inhibited or suppressed apoptosis, especially the neoplasms that are associated with oncogenic forms of ABL, *e.g.*, CML and ALL.

The oncogenic diseases to be treated by the compounds provided by the present invention can be at the accelerated, blast, or chronic stage. The compounds of the present invention can also be used to prevent relapse in patients treated with interferon-$\alpha$, bone marrow transplantation, or other therapeutic modulates.

One skilled in the art will recognize that a therapeutically effective amount of the protein kinase C inhibitor of the present invention is the amount sufficient to induce apoptosis or cell death and that this amount varies *inter alia,* depending upon the size, the type of the neoplasia, the concentration of the compound in the therapeutic formulation, and the condition of the patient. Both *in vivo* and *in vitro* tests can be used to determine the amount of a compound needed for inducing apoptosis. For example, hematopoietic cells expressing either the v-ABL or BRC-ABL can be exposed to conditions that normally induce apoptosis of nontransformed hematopoietic cells, *e.g.*, interleukin-3 removal from cells dependent upon interleukin-3 for survival (Owen P., et al., 1993. J Biol Chem 268: 15696; Owen P., et al., 1993. J Biol Chem 268: 15696; Evans C., et al., 1995. J Cell Sci 108: 2591). Cells expressing the activated v-ABL do not undergo apoptosis under these circumstances. Upon administering the compounds provided by the present invention, apoptosis or cell death can be measured by any means known in the art. Cell death can be determined and quantified via trypan blue exclusion, and reduced clonogenecity in soft agar.

Generally, an amount of protein kinase C inhibitor to be administered as a therapeutic agent for treating neoplasms associated with oncogenic forms of ABL, *e.g.*, CML and ALL will be determined on a case by case basis by an attending physician. As a guideline, the extent of the neoplasia, the body weight and age of the patient will be considered when setting an appropriate dose.

Generally, a suitable dose is one that results in a concentration of the protein kinase C inhibitor at the treatment site in the range of 0.5 nM to 200 $\mu$M, and more usually 20 nM to 80 nM. It is expected that a dose which provide a serum concentration of 0.5 nM to 10 nM should be sufficient in most circumstances.

To obtain these treatment concentrations, a patient in need of treatment likely will be administered between about 0.5 mg per day per kg of body weight and 1.5 mg per day per kg. Usually, not more than about 1.0 mg per day per kg of body weight of protein kinase C inhibitor should be needed. As noted above, the above amounts may vary on a case-by-case basis.

The compounds of formula I and the preferred compounds of formula Ia and Ib are preferably formulated prior to administration. Suitable pharmaceutical formulations are prepared by known procedures using well known and readily available ingredients. In making the compositions suitable for use in the method of the present invention, the active ingredient will usually be mixed with a carrier, or diluted by a carrier, or enclosed within a carrier which may be in the form of a capsule, sachet, paper or other container. When the carrier serves as a diluent, it may be a solid, semisolid or liquid material which acts as a vehicle, excipient or medium for the active ingredient. Thus, the compositions can be in the form of tablets, pills, powders, lozenges, sachets, cachets, elixirs, suspensions, emulsions, solutions, syrups, aerosol (as a solid or in a liquid medium), soft and hard gelatin capsules, suppositories, sterile injectable solutions and sterile packaged powders for either oral or topical application.

Some examples of suitable carriers, excipient, and diluents include lactose, dextrose, sucrose sorbitol, mannitol, starches, gum acacia, calcium phosphates, alginate, tragacanth, gelatin, calcium silicate, microcrystalline cellulose, polyvinylpyrrolidone, cellulose, water syrup, methyl cellulose, methyl and propylhydroxybenzoates, talc, magnesium stearate and mineral oil. The formulations can additionally include lubricating agents, wetting agents, emulsifying and suspending agents, preserving agents, sweetening agents or flavoring agents. The compositions of the invention may be formulated so as to provide quick, sustained or delayed release of the active ingredient after administration to the patient. The compositions are preferably formulated in a unit dosage form, each dosage containing from about 0.05 mg to about 3 g, more usually about 64 mg of the active ingredient. However, it will be understood that the therapeutic dosage administered will be determined by the physician in the light of the relevant circumstances including the severity of the condition to be treated, the choice of compound to be administered and the chosen route of administration. Therefore, the above dosage ranges are not intended to limit the scope of the invention in any way. The term

"unit dosage form" refers to physically discrete units suitable as unitary dosages for human subjects and other mammals, each unit containing a predetermined quantity of active material calculated to produce the desired therapeutic effect, in association with a suitable pharmaceutical carrier.

In addition to the above formulations, most of which may be administered orally, the compounds used in the method of the present invention also may be administered topically. Topical formulations include ointments, creams and gels.

Ointments generally are prepared using either (1) an oleaginous base, i.e., one consisting of fixed oils or hydrocarbons, such as white petrolatum or mineral oil, or (2) an absorbent base, i.e., one consisting of an anhydrous substance or substances which can absorb water, for example anhydrous lanolin. Customarily, following formation of the base, whether oleaginous or absorbent, the active ingredient (compound) is added to an amount affording the desired concentration.

Creams are oil/water emulsions. They consist of an oil phase (internal phase), comprising typically fixed oils, hydrocarbons, and the like, such as waxes, petrolatum, mineral oil, and the like, and an aqueous phase (continuous phase), comprising water and any water-soluble substances, such as added salts. The two phases are stabilized by use of an emulsifying agent, for example, a surface active agent, such as sodium lauryl sulfate; hydrophilic colloids, such as acacia colloidal clays, veegum, and the like. Upon formation of the emulsion, the active ingredient (compound) customarily is added in an amount to achieve the desired concentration.

Gels comprise a base selected from an oleaginous base, water, or an emulsion-suspension base. To the base is added a gelling agent which forms a matrix in the base, increasing its viscosity. Examples of gelling agents are hydroxypropyl cellulose, acrylic acid polymers, and the like. Customarily, the active ingredient (compounds) is added to the formulation at the desired concentration at a point preceding addition of the gelling agent.

The amount of compound incorporated into a topical formulation is not critical; the concentration should be within a range sufficient to permit ready application of the formulation to the affected tissue area in an amount which will deliver the desired amount of compound to the desired treatment site.

The customary amount of a topical formulation to be applied to an affected tissue will depend upon an affected tissue size and concentration of compound in the formulation. Generally, the formulation will be applied to the effected tissue in an amount affording from about 1 to about 500 µg compound per $cm^2$ of an affected tissue. Preferably, the applied amount of compound will range from about 30 to about 300 µg/cm, more preferably, from about 50 to about 200 $\mu g/cm^2$, and, most preferably, from about 60 to about 100 $\mu g/cm^2$.

The following formulation examples are illustrative only and are not intended to limit the scope of the invention in any way.

Formulation 1

Hard gelatin capsules are prepared using the following ingredients:

| | Quantity (mg/capsule) |
|---|---|
| Active agent | 250 |
| starch, dried | 200 |
| magnesium stearate | 10 |
| Total | 460 mg |

The above ingredients are mixed and filled into hard gelatin capsules in 460 mg quantities.

Formulation 2

A tablet is prepared using the ingredients below:

| | Quantity (mg/capsule) |
|---|---|
| Active agent | 250 |

(continued)

| | Quantity (mg/capsule) |
|---|---|
| cellulose, microcrystalline | 400 |
| silicon dioxide, fumed | 10 |
| stearic acid | 5 |
| Total | 665 mg |

The components are blended and compressed to form tablets each weighing 665 mg.

Formulation 3

Tablets each containing 60 mg of active ingredient are made as follows:

| | Quantity (mg/tablet) |
|---|---|
| Active agent | 60 mg |
| starch | 45 mg |
| microcrystalline cellulose | 35 mg |
| polyvinylpyrrolidone (as 10% solution in water) | 4 mg |
| sodium carboxymethyl starch | 4.5 mg |
| magnesium stearate | 0.5 mg |
| talc | 1 mg |
| Total | 150 mg |

The active ingredient, starch and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°C and passed through a No. 18 mesh U.S. sieve. The sodium carboxymethyl starch, magnesium stearate and talc, previously passed through a No. 60 mesh U.S. sieve, are then added to the granules which, after mixing, are compressed on a tablet machine to yield tablets each weighing 150 mg.

The principles, preferred embodiments and modes of operation of the present invention have been described in the foregoing specification. The invention which is intended to be protected herein, however, is not to be construed as limited to the particular forms disclosed, since they are to be regarded as illustrative rather than restrictive. Variations and changes may be made by those skilled in the art without departing from the spirit of the invention.

**Claims**

1. The use of an inhibitor of the β isozyme of protein kinase C wherein the inhibitor is isozyme selective and where the isozyme selectivity is selected from the group consisting of beta-1 and beta-2 isozymes in the preparation of a medicament for treating a neoplasm, chronic myeloidleukemia or acute lymphoid leukaemia associated with an oncogenic form of ABL.

2. The use of claim 1 wherein the inhibitor of the β isozyme of protein kinase C is a bis-indolylmaleimide or a macrocyclic bis-indolylmaleimide.

3. The use of claim 2 wherein the protein kinase C inhibitor has the following formula:

(I)

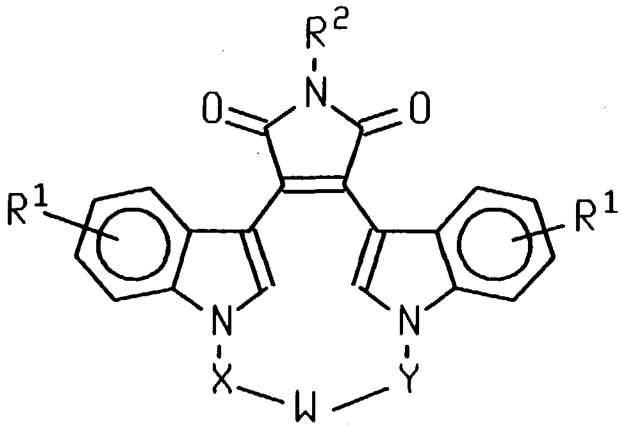

wherein:

W is -O-, -S-, -SO-, $-SO_2-$, -CO-, $C_2$-$C_6$ alkylene, substituted alkylene, $C_2$-$C_6$ alkenylene, -aryl-, $-aryl(CH_2)_mO-$, -heterocycle-, $-heterocycle-(CH_2)_mO-$, -fused bicyclic-, $-fused\ bicyclic-(CH_2)_mO-$, $-NR^3-$, $-NOR^3-$, -CONH-, or -NHCO-;
X and Y are independently $C_1$-$C_4$ alkylene, substituted alkylene, or together X, Y, and W combine to form $-(CH_2)_n$-AA-;
$R^1$s are hydrogen or up to four optional substituents independently selected from halo, $C_1$-$C_4$ alkyl, hydroxy, $C_1$-$C_4$ alkoxy, haloalkyl, nitro, $NR^4R^5$, or - NHCO($C_1$-$C_4$ alkyl);
$R^2$ is hydrogen, $CH_3CO$-, $NH_2$, or hydroxy;
$R^3$ is hydrogen, $(CH_2)_m$aryl, $C_1$-$C_4$ alkyl, -COO($C_1$-$C_4$ alkyl), $-CONR^4R^5$, - (C=NH)$NH_2$, -SO($C_1$-$C_4$ alkyl), - $SO_2$ ($NR^4R^5$), or $-SO_2$ ($C_1$-$C_4$ alkyl);
$R^4$ and $R^5$ are independently hydrogen, $C_1$-$C_4$ alkyl, phenyl, benzyl, or combine to the nitrogen to which they are bonded to form a saturated or unsaturated 5 or 6 member ring;
AA is an amino acid residue;
m is independently 0, 1, 2, or 3; and
n is independently 2, 3, 4, or 5.

**4.** The use of claim 3 wherein the protein kinase C inhibitor has the following formula:

(Ib)

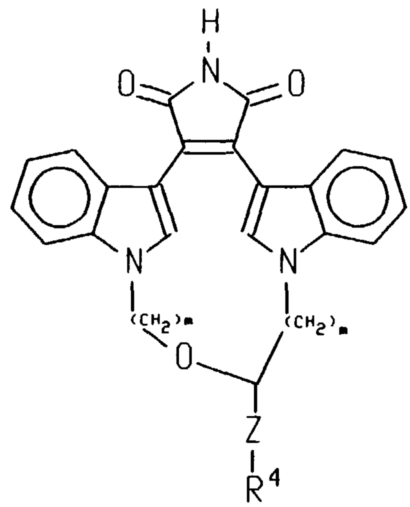

wherein Z is $-(CH_2)_p$- or $-(CH_2)_p-O-(CH_2)_p$-; $R^4$ is hydroxy, -SH, $C_1$-$C_4$ alkyl, $(CH_2)_m$aryl, -NH(aryl), $-N(CH_3)$ $(CF_3)$, $-NH(CF_3)$, or $-NR^5R^6$; $R^5$ is hydrogen or $C_1$-$C_4$ alky; $R^6$ is hydrogen, $C_1$-$C_4$ alkyl or benzyl; p is 0, 1, or 2; and m is independently 2 or 3.

5. The use of claim 3 wherein the protein kinase C inhibitor has the following formula:

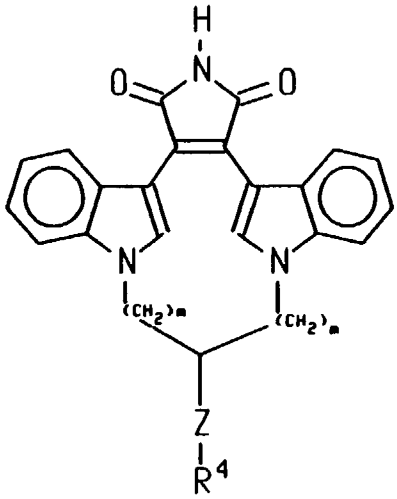

(Ia)

wherein Z is $-(CH_2)_p-$; $R^4$ is $-NR^5R^6$, $-NH(CF_3)$, or $-N(CH_3)(CF_3)$; $R^5$ and $R^6$ are independently H or $C_1$-$C_4$ alkyl; p is 0, 1, or 2; and m is independently 2 or 3.

6. The use of claim 4, wherein the protein kinase C inhibitor comprises (S)-3,4-[N,N'-1,1'((2''-ethoxy)-3'''(O)-4'''-(N-dimethylamino)-butane)-bis-(3,3'-indolyl)]-1(H)-pyrrole-2,5-dione or its acid salt.

7. The use according to any preceding claim, wherein the oncogenic form of ABL is caused by chromosome rearrangement.

8. The use according to any preceding claim, wherein the medicament is for the treatment of mycloid leukemia and the oncogenic form of ABL is BRC-ABL.

9. The use of an inhibitor of the β isozyme of protein kinase C wherein the inhibitor is isozyme selective and where the isozyme selectivity is selected from the group consisting of beta-1 and beta-2 isozymes in the preparation of a medicament for inducing apoptosis in cells.

10. The use according to claim 9 wherein the inhibitor of the β isozyme of protein kinase C is a bis-indolylmaleimide or a macrocyclic bis-indolymaleimide.

European Patent Office

# PARTIAL EUROPEAN SEARCH REPORT

which under Rule 45 of the European Patent Convention shall be considered, for the purposes of subsequent proceedings, as the European search report

Application Number
EP 98 30 9616

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,D | US 5 552 396 A (MCDONALD III JOHN H ET AL) 3 September 1996<br>* column 1, line 59-64; example 5 *<br>--- | 1-21 | A61K31/40 |
| X | OSADA, HIROYUKI ET AL: "A new inhibitor of protein kinase C, RK-1409 (7-oxostaurosporine). I. Taxonomy and biological activity"<br>J. ANTIBIOT. (1992), 45(2), 189-94 CODEN: JANTAJ;ISSN: 0021-8820, XP002104954<br>* abstract *<br>* page 191 - page 193; figures 3,5 *<br>--- | 1,2,7-9, 14-21 | |
| X | MIYAMOTO, KENICHI ET AL: "Effect of staurosporine derivatives on protein kinase activity and vinblastine accumulation in mouse leukemia P388/ADR cells"<br>J. PHARM. PHARMACOL. (1993), 45(1), 43-7 CODEN: JPPMAB;ISSN: 0022-3573, XP002104955<br>* abstract; figure 3; tables 1-3 *<br>----- | 1,2,7-9, 14-21 | |

TECHNICAL FIELDS SEARCHED (Int.Cl.6)

A61K

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 8 June 1999 | A. Jakobs |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 98 30 9616

Although claims 1-21 are directed to a method of treatment of the human/animal body (Article 52(4) EPC), the search has been carried out and based on the alleged effects of the compound/composition.

---

Claim(s) searched completely:
3-6,10-12

Claim(s) searched incompletely:
1,2,7-9,14-21

Reason for the limitation of the search:

In view of the large number of compounds, which are defined by the general definition(s)/formulae used in claims 1,2,7-9,14-21, the search had to be restricted. The search was limited to the compounds for which pharmacological data was given and / or the compounds mentioned in the claims, and to the general idea underlying the application.